Europäisches Patentamt

(19) European Patent Office    (11) Publication number: **0 041 752**

Office européen des brevets    **B1**

(12) **EUROPEAN PATENT SPECIFICATION**

(45) Date of publication of patent specification: **17.10.84**    (51) Int. Cl.³: **A 61 B 6/02,** A 61 B 6/04, A 61 B 6/00

(21) Application number: **81200579.1**

(22) Date of filing: **29.05.81**

(54) **Radiography apparatus incorporating image subtraction.**

(30) Priority: **09.06.80 NL 8003354**

(43) Date of publication of application:
**16.12.81 Bulletin 81/50**

(45) Publication of the grant of the patent:
**17.10.84 Bulletin 84/42**

(84) Designated Contracting States:
**DE FR GB NL**

(56) References cited:
**EP-A-0 022 722**
**DE-A-2 343 162**
**FR-A-2 239 975**
**FR-A-2 260 251**
**FR-A-2 345 983**
**FR-A-2 387 634**
**US-A-3 101 407**
**US-A-3 894 181**
**US-A-4 029 963**
**US-A-4 053 779**

(73) Proprietor: **N.V. Philips' Gloeilampenfabrieken**
**Groenewoudseweg 1**
**NL-5621 BA Eindhoven (NL)**

(72) Inventor: **Zonneveld, Frans Wessel**
**INT. OCTROOIBUREAU B.V. Prof. Holstlaan 6**
**NL-5656 AA Eindhoven (NL)**

(74) Representative: **Scheele, Edial François et al**
**INTERNATIONAAL OCTROOIBUREAU B.V. Prof.**
**Holstlaan 6**
**NL-5656 AA Eindhoven (NL)**

Courier Press, Leamington Spa, England.

## Description

The invention relates to a radiography apparatus, comprising a detector and a patient table which is arranged to be translatable with respect to either the detector on its own or to the detector together with a radiation source and a subtraction device for forming difference images from sub-images respectively to be recorded, which sub-images differ in respect of at least one image-determining parameter. Such an apparatus is known from US—A— 3 894 181.

For examinations such as for the angiography it is desirable to obtain an image in which blood vessels are reproduced in an accurately localized manner and with high contrast. Known apparatus applicable for angiographic such as described in US—A— 3,894,181 do not satisfy this requirement, because the contrast resolution therein can become too low, which is *inter alia* due to the fact that intensifier screens or television camera tubes are used for recording the radiographic image. In apparatus of this kind, an instantaneous shadow image is made of a part of a patient under examination. The instant of recording the image should be suitably related to the instant of administration of, for example, a contrast medium, whereby a mean propagation rate through the body is assumed. Some parts of the object will consequently not yet contain a sufficient amount of contrast medium, while the concentration will already have decreased in other parts. The drawbacks thereof can be eliminated only by administering a comparatively large amount of contrast medium; obviously, this would be detrimental to the patient. This drawback may be even more serious when images are formed by means of a radioactive tracer.

The invention has for its object to mitigate these drawbacks; to this end, an apparatus of the described kind in accordance with the invention is characterized in that for controlling the translation of the patient table with respect to the detector there is provided an automatic speed control device which can be controlled as a function of the progress of an image-determining substance administered to the patient and in that the detector is composed of a series of individually readable detector elements.

Because high contrast sub-images can be formed in an apparatus in accordance with the invention, it is possible to form by subtraction an image in which, for example, the vascular system is reproduced with high contrast. A scanogram recorded by means of a device as described in DE—A—26 13 809 has substantially higher contrast resolution than customary shadow images. When scanograms of this kind from several directions are combined, a three-dimensional image matrix can be formed in which the blood vessels are represented in space. Because merely the presence or absence of a blood vessel has to be discriminated, in comparison with a scan-derived image only a limited number of profiles are required and the image reconstruction can be realized in an extremely simple manner. For an orientation within the body, it may be advantageous to leave the subtraction incomplete, so that structures of the body, for example, suitable bones, remain visible for reference purposes.

A preferred embodiment of the apparatus comprises means for transferring information from scan-derived images, having a high resolution, to scanograms. The possibility of accurate (re)positioning of the patient also enables relationships to be established between scan-derived images and profiles for scanograms. From a combination of a series of scanograms of different orientation, image elements in which a blood vessel is present can thus be indicated in the scan-derived images.

For the formation of a scanogram after the administration of a contrast medium in a preferred embodiment, the speed of translation of the patient table with respect to the detector is adapted to the propagation rate of the contrast medium in the organs to be reresented. As a result, measurement always takes place in sectional regions in which the instantaneous concentration of the contrast medium is highest, so that the dose thereof may be lower. The speed of the translation, and hence the propagation rate of the contrast medium; can aso be used for kymographic reproduction in angiography.

For medical diagnosis it is often desirable to obtain images having a high resolution in time. To achieve this, a preferred embodiment in accordance with the invention comprises means for the kinematographic representation of subtraction images which have been measured in a fixed position of the object with respect to the detector. When a series of profiles are thus sequentially recorded over a period of time which extends from at least a few exposures before penetration of, for example, a contrast medium until several exposures after the instant of maximum concentration thereof in the body section, and when the images without contrast medium are subtracted from images with contrast medium and the images thus formed are kinematographically presented, an image with high resolution in time is obtained. This device enables recording to be effected with a frequency of, for example, more than 100 exposures per second, while the frequency in the case of dynamic scanograms, i.e. images of a part of a patient which is displaced along the source/detector unit during the exposures, is of the order of at the most one image every two seconds. For the exposures, use can be made of said contrast medium for X-ray shadow graph images and/or of a radioactive tracer for emission images.

In a preferred embodiment, the detector is rotatable, either on its own or together with a radiation source, about the patient table, so that profiles can be formed from different orientations. Thus, when a series of profiles are made under the same circumstances, except for the radial orientation, scan-derived profile images can be formed from each time a corresponding one of the series of scan-derived profile images, as known from the X-ray scanning method, and these scan-derived profile images can be kinematographically presented. Thus, a film is made in which the variation of the contrast in time is reproduced in a scan-derived profile image again with the high resolution in time of at least 100 images per second.

A further preferred embodiment in accordance with the invention includes a control device for starting the recordings which is controlled by the instant of injection of the contrast medium or by the heart beat or by both. For recordings in parts of a patient where the heart beat does not have a disturbing effect, a coupling between the instant of injection and the start of the recordings suffices. On the basis of known propagation rates of the contrast medium or the radioactive tracer through relevant parts of the body of the patient, the time difference between injection and the start of the recordings can be optimized. For recordings of regions in which the heart beat could have a disturbing effect, synchronization with the heart beat is employed for adjusting the starting instant. Notably when the vascular system is to be examined, it may be advantageous also to relate the injection to the heart beat.

For determining the recording frequency, a control device in a further embodiment can be controlled as a function of the rate of change of the concentration of the contrast medium in the relevant part of the body. The variation of the concentration thereof is known from experience for many organs or parts of the body. In the case of rapid variations of contrast, images can be recorded at a high frequency, while the frequency can be reduced during an initial (starting) phase and notably during a terminal phase. The radiation dose for the patient may thus be reduced. For moving image representation intermediate images can be formed by interpolation to supply the missing image frames in the kinematographic representation which are caused by the reduction in the exposure frequency.

In a preferred embodiment in accordance with the invention, the energy, i.e. the frequency of the imaging radiation, serves as the variable quantity in recording the images to be subtracted. To achieve this, use can be made of an X-ray source which is suitable for operation at different high voltages. It is to be noted that this method is known *per se* from United States Patent Specification 3,894,181 where, however, the described limitation of the resolution occurs.

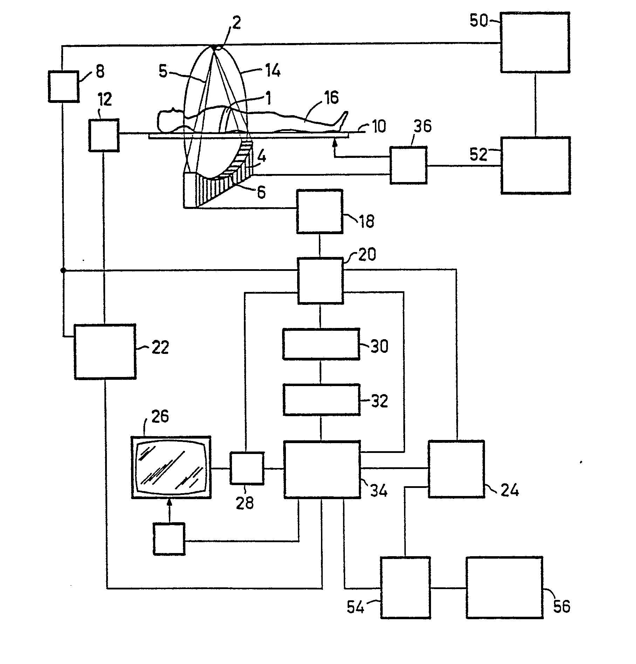

Some preferred embodiments in accordance with the invention will be described in detail hereinafter with reference to the drawing. An X-ray scanner which is diagrammatically shown in the drawing comprises an X-ray source 2, a detector 4 which is composed of a series of detector elements 6 which are adjacently mounted in a row and which may alternatively form a closed ring so that the detector need not be rotatable about an object 16, a high voltage generator 8 and an object carrier 10 with a drive mechanism 12. The source 2 and the detector 4 are mounted on a common support 14 which enables the source and the detector to be rotated about the object 16 positioned on the object carrier. For reading the detector signals, the apparatus comprises a detector read-out unit 18 which preferably includes a signal amplifier and an analog-to-digital converter. Under the control of processed detector signals, a control device 20 controls a control mechanism 22 which controls and effects the rotation of the source/detector support, the translation of the object carrier and the control of the high-voltage generator 8. The control device 20 is furthermore connected to the memory 24 and to a signal modulator 28 associated with a television monitor 26. For further signal processing there are provided an arithmetic unit 30, a selection device 32 and a reconstruction device 34.

This apparatus enables a scan-derived sectional image to be formed and recorded at each of a succession of positions of the object carrier. All relevant measurment signals are processed in the detector read-out unit 18 and are applied, *via* the control device 20 for synchronisation, to the arithmetic unit 30 for further processing, or are stored in the memory 24 for later processing. A series of successive scan-derived sectional images are used to form a three-dimensional matrix of absorbtion values from which a scan-derived image can be formed and displayed on the monitor in known manner. In a manner similar to the method described in the United States Patent Specification 3,101,407, scanograms can be recorded by means of the apparatus by sliding the object carrier (preferably step-wise) through the support 14 whilst the rotation device is locked. A location indicator 36, for example, as described in DE—A—26 13 809, may be of assistance for locating each of the scan-derived sectional images with respect to a reference plane. The device furthermore comprises a subtraction device whereby scanograms can be subtracted image-element-wise. When a scanogram of a given zone without contrast medium is subtracted from a scanogram of the same zone with a contrast medium which has been recorded at a later stage, an image is formed in which in principle only the contrast medium is reproduced. For the formation of a stereoscopic pair, two corresponding pairs of scanograms can be recorded from different directions, followed by appropriate image subtraction. From pair-wise recordings from serveral directions, a three-dimensional matrix can be reconstructed in which there is provided an indication in respect of each image element as to whether or not, for example, a blood vessel is present therein. Because blood vessels have a simple cross-section and because this cross-section is comparatively small and because, moreover, it is only necessary to discriminate with respect to the presence or absence of a blood vessel by means of simple threshold criteria, the process of computed image reconstruction may be simple and a comparatively small number of orientations will suffice for the irradiation. This matrix of image elements can be related to scan-derived images; for example, the blood vessels may be drawn in a scan-derived image in an exact position. As a result, a direct relationship is possible between the high-quality information in a scan-derived image and the location of a blood vessel therein. When this device is extended to include a system for measuring emission images in which the contrast is provided by the use of a radioactive tracer, shadow images and emission images can be correlated and combined to form an image with an optimum information content. Preferably, the same patient table is used for both recordings, so that no positioning problems would be encountered. It is also possible to use an apparatus in which both X-rays and gamma radiation can be detected.

A further embodiment of the device comprises a control device 50 whereby the X-ray source can be pulsed with a frequency of up to, for example, 150 pulses per second and which is connected to a synchronization device 52 which supplies the control device 50 with trigger signals which are related to the heart beat of a patient positioned on the patient table. The reconstruction device 34 comprises a subtraction device 54 which is connected to a kinematographic display device 56 and which can also receive information from the memory 24. The patient table 10 is arranged within the support 14 for the X-ray source and the detector so that a body section 1 of a patient 16 under examination on the patient table, is situated within a fan-shaped flat X-ray beam 5, after which the rotation of the support 14 is locked. Using an injection device (not shown), which can be controlled by the synchronization device 52, a contrast medium is injected and the recording of profiles commences in response to a signal from the control device 50 which can also be given a certain time before the injection. The local path absorption values of each exposure are recorded by means of the detection elements, and from a succession of these values a scan-derived profile image is formed which can be stored in the memory 24. Preferably, a mean scan-derived profile image is formed from successive scan-derived profile images in order to minimize changes, apart from the presence or absence of the contrast medium. This mean scan-derived profile image is each time subtracted from the last recorded scan-derived profile image. The averaging is stopped either at the instant of injection or after a given row of subtraction images. The last mean image is used as a basis for further subtraction. A similar procedure can be followed for a number of radial orientations of the source/detection system with respect to the patient. It is desirable that each of these series is recorded under closely similar circumstances, such as the same time relationship with respect to the heart beat and the same quantity of contrast medium which must be negligibly small with respect to the amount of blood notably in the case of angiography. The latter requirement is satisfied by the fact that background correction is in effect performed by the averaging and the subtraction processes. A suitable correlation with the heart beat for each of the series prevents disturbances due to movement of the heart. The number of recordings per unit of time can be chosen for each of the series, as a function of the rate of change of the concentration which is usually known from experience or which can otherwise be detected. Scan images formed with each time one profile per series which corresponds in exposure time can be reproduced as a cine film when linked; this film in the form of scan-derived profile images again exhibits the higher resolution in time up to, for example, 150 images per second. The contrast resolution is then determined by the number of series, and hence by the number of projection directions and the accuracy of the image reconstruction from the profiles. However, usually the information whether or not, for example, a blood vessel is present in a given location will be sufficient. In that case a limited number of series suffices, and therefore a low radiation dose, and a simple, and fast, reconstruction will suffice. If dimensions, for example, the diameter of a blood vessel or the heart volume or the maximum difference in heart volume taken over a heart beat, are important for an examination, several series can be recorded and reconstructed. By slection of Hounsfield values, for which appropriate filters may be used, structures having a given radiation absorption value can be reproduced in the images, or structures outside a given absorption range can be removed. As a result, a discrimination can be performed as regards contrast medium concentration so that, for example, blood vessels in the image can be enhanced or the vascular system or any other organism to be injected can be correlated to a tissue having a given absorption value. For example, the vascular system in the lungs can become localized in the long tissue. Apart from the adaptation of the detector elements to the nature of the radiation, a radiography apparatus in accordance with the invention for performing emission measurements by injection of a radioactive tracer has a similar construction, and the procedure may also be exactly the same.

In the preferred embodiment for measuring by means of two radiation beams of mutually different energy, the X-ray generator is formed, for example, by an "Optimus 200" which is marketed by Philips and which comprises an X-ray tube which enables operation with radiation of, for example, 90 kV and 140 kV. For reducing a resultant large difference in the level of the measurement signals, the pulse duration can be chosen to be different for the various wavelengths, for example, 1 ms for 140 kV and 2 ms for 90 kV. Measurements performed with these different wavelengths produce image information in which, for example, suitable chosen contrast media occur to a different degree. By subtraction of these images, therefore, an image can again be formed in which notably the contrast medium is clearly shown. In order to neutralize excursions of or in the patient as much as possible, the two images to be mutually processed are preferably recorded as quick in sucession as possible. To achieve this, the source can be alternately pulsed with the two high voltages for a scan image and the measurements with each of the high voltages can be combined, and subsequently subtracted. For the formation of scanograms, two different scan-derived profile images are formed in each position of the source and the detector with respect to the patient. Images thus formed can be combined in the described manner, after which they are displayed or recorded. An image formed by means of the high-energy radiation beam also provides information as regards the electron density in the object. This information can be directly used for radiotherapy.

## Claims

1. A radiography apparatus comprising, a detector and a patient table which is arranged to be translatable with respect to either the detector on its own or to the detector together with a radiation source, and a subtraction device for forming difference images from sub-images respectively to be recorded, which sub-images differ in respect of at least one image-determining parameter, characterized in that for controlling the translation of the patient table with respect to the detector there is provided an automatic speed control device which can be controlled as a function of the progress of an image-determining substance administered to the patient and in that the detector is composed of a series of individually readable detector elements.

2. A radiography apparatus as claimed in Claim 1, characterized in that there is provided a control device for controlling the frequency of scan-derived profile images to be recorded as a function of the concentration variation of a contrast medium in a part of a patient under examination.

3. A radiography apparatus as claimed in Claim 1, characterized in that it includes an X-ray source with a high voltage generator for respectively generating in alternation radiation of two different wavelengths which high voltage generator is constructed to adapt pulse durations of the alternating actuation of the source to a degree of absorption of the respectively generated radiation.

## Revendications

1. Appareil radiographique comportant un détecteur et une table d'examen disposée à translation par rapport au détecteur ou à l'ensemble du détecteur et de la source de radiation ainsi qu'un dispositif de soustraction servant à former des images différentielles à partir de sous-images respectivement à enregistrer, sous-images qui diffèrent à l'égard d'au moins un paramètre déterminant l'image, caractérisé en ce que pour la commande de la translation de la table d'examen par rapport au détecteur, il est prévu un dispositif de commande automatique de la vitesse qui peut être commandé en fonction de la progression d'une substance de détermination d'image administrée au patient et en ce que le détecteur est constitué d'une série d'éléments détecteurs pouvant être lus individuellement.

2. Appareil radiographique selon la revendication 1, caractérisé en ce qu'il est prévu un dispositif de commande servant à commander la fréquence d'images de profil dérivées du balayage et devant être enregistrées en fonction de la variation de la concentration d'une substance de contraste dans une partie d'un patient soumis à l'examen.

3. Appareil radiographique selon la revendication 1, caractérisé en ce qu'il comporte une source de rayons X munie d'un générateur haute tension servant à engendrer à alternation respectivement des radiations de deux longueurs d'onde différentes, générateur haute tension qui a été conçu pour adapter les durées d'impulsion de l'actionnement alterné de la source à un degré d'absorption de la radiation respectivement engendrée.

## Patentansprüche

1. Röntgengerät mit einem Detektor und einem Patientenuntersuchungstisch, der derart angeordnet ist, dass er in bezug entweder auf den Detektor allein oder auf den Detektor zusammen mit einer Strahlungsquelle translatorisch bewegbar ist, und mit einer Subtraktionseinrichtung zur Bildung von Differenzbildern aus Unterbildern, die aufzunehmen und in bezug auf zumindest eine bildbestimmende Grösse verschieden sind, dadurch gekennzeichnet, dass zur Steuerung der Translationsbewegung des Patientenuntersuchungstisches in bezug auf den Detektor ein automatisches Geschwindigkeitssteuergerät vorgesehen ist, das abhängig von der Ausbreitung einer bildbestimmenden, dem Patienten verabreichten Substanz steuerbar ist, und dass der Detektor aus einer Reihe einzeln lesbarer Detektorelemente besteht.

2. Röntgengerät nach Anspruch 1, dadurch gekennzeichnet, dass ein Steuergerät zur Steuerung der Frequenz der durch Abtastung erhaltenen Profilbilder vorgesehen ist, die in Abhängigkeit von der Konzentrationsvariation eines Kontrastmittels in einem Teil eines zu untersuchenden Patienten aufzuzeichnen sind.

3. Röntgengerät nach Anspruch 1, dadurch gekennzeichnet, dass darin eine Röntgenstrahlungsquelle mit einem Hochspannungsgenerator zum abwechselnden Erzeugen von Strahlung mit zwei verschiedenen Wellenlängen vorgesehen ist, und dass der Hochspannungsgenerator so ausgelegt ist, dass die Impulsdauer der abwechselnden Quellenaktivierung an die Absorption der jeweils erzeugten Strahlung angepasst ist.